# EUROPEAN PATENT APPLICATION

(11) **EP 4 830 751 A1**
(43) Date of publication of application: **28.10.2026**
(21) Application number: 25172649.3
(22) Date of filing: 25.04.2025
(51) Int. Cl.: A61M 11/00, A61M 13/00

(54) **SURGICAL DEVICE**

(71) Applicant: PROVIEW-MBD BIOTECH CO., LTD., Taipei City (TW)
(72) Inventor: CHANG, Yu-Chia, Taipei City (TW); HSU, Nai-Sheng, Taoyuan City (TW); KU, Wen-Hua, Baoshan Township, Hsinchu County 308005 (TW); TSAI, Hsieh-Chih, Taipei City 106018 (TW); LIN, Shuian-Yin, Zhubei City, Hsinchu County 302053 (TW); KUO, Jer-Cheng, Taipei City (TW)
(74) Representative: dompatent

(57) **Abstract**

A surgical device configured to spray a flowable material cooled by a coolant and including a syringe, a first inner tube, an outer tube and a container. The syringe has an outlet and is configured to accommodate the flowable material. The first inner tube is disposed on an end of the syringe and in fluid communication with the outlet. The outer tube is disposed on the syringe and surrounds the first inner tube. A cooling channel is formed between the first inner tube and the outer tube. The container is in fluid communication with the cooling channel and configured to accommodate the coolant.

## Description

### Technical Field

The disclosure relates to a surgical device, more particularly to a surgical device configured to spray a flowable material cooled by a coolant.

### Background

Thermosensitive hydrogel typically maintains liquid properties at a low temperature and transitions to a solid gel at human body temperature, allowing for easy application and excellent efficacy of adhesion prevention. Thus, during open or minimally invasive surgeries in which the tissue undergoes trauma, the thermosensitive hydrogel is commonly used as an anti-adhesion material to prevent or reduce post-surgical tissue adhesions.

However, thermosensitive hydrogels require a constant low temperature to maintain fluidity. Thus, conventionally, the thermosensitive hydrogels are stored in a medical refrigerator before the surgery. When the surgery starts, the thermosensitive hydrogels are taken out of the refrigerator, and the surgery is required to be performed as soon as possible before the temperature of the thermosensitive hydrogels is significantly increased. Such limitation causes inconvenience for spraying thermosensitive hydrogels during open and minimally invasive surgery.

Furthermore, in specific minimally invasive surgical applications, particularly laparoscopic procedures, said thermosensitive hydrogels must be delivered through an environment maintained at physiological temperature for an extended duration of administration, thereby necessitating implementation of a cooling mechanism to preserve the fluid state of said hydrogels.

### SUMMARY

The disclosure provides a surgical device that facilitates the spraying of a flowable material.

One embodiment of this disclosure provides a surgical device configured to spray a flowable material cooled by a coolant and including a syringe, a first inner tube, an outer tube and a container. The syringe has an outlet and is configured to accommodate the flowable material. The first inner tube is disposed on an end of the syringe and in fluid communication with the outlet. The outer tube is disposed on the syringe and surrounds the first inner tube. A cooling channel is formed between the first inner tube and the outer tube. The container is in fluid communication with the cooling channel and configured to accommodate the coolant.

According to the surgical device discussed above, the cooling channel in fluid communication with the container is formed between the first inner tube and the outer tube, and the first inner tube is in fluid communication with the outlet of the syringe. Thus, the coolant in the container is allowed to flow into the cooling channel to cool the flowable material flowing into the first inner tube from the outlet of the syringe. In this way, the flowable material is cooled to a constant low temperature to maintain fluidity, thereby facilitating the spraying of the flowable material.

### BRIEF DESCRIPTION OF THE DRAWINGS

The present disclosure will become better understood from the detailed description given herein below and the accompanying drawings which are given by way of illustration only and thus are not intending to limit the present disclosure and wherein:
FIG. 1 is a perspective view of a surgical device according to a first embodiment of the disclosure;
FIG. 2 is an exploded view of the surgical device in FIG. 1;
FIG. 3 is a top cross-sectional view of the surgical device in FIG. 1;
FIG. 4 is a partially enlarged side cross-sectional view of the surgical device in FIG. 1;
FIG. 5 is a side cross-sectional view showing a mixing portion of the surgical device in FIG. 1; and
FIGS. 6 and 7 are a side cross-sectional views of a surgical device according to a second embodiment of the disclosure.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

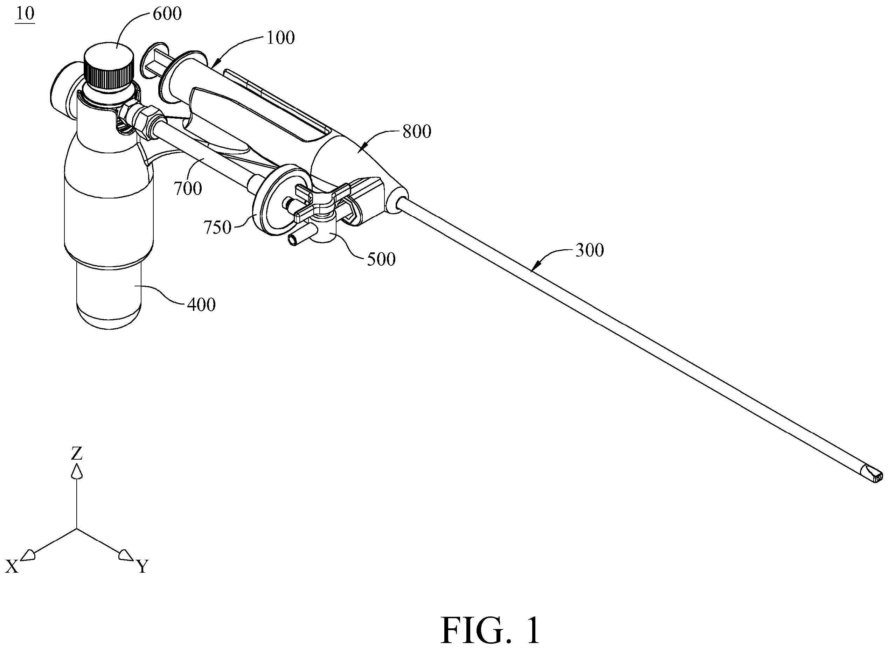

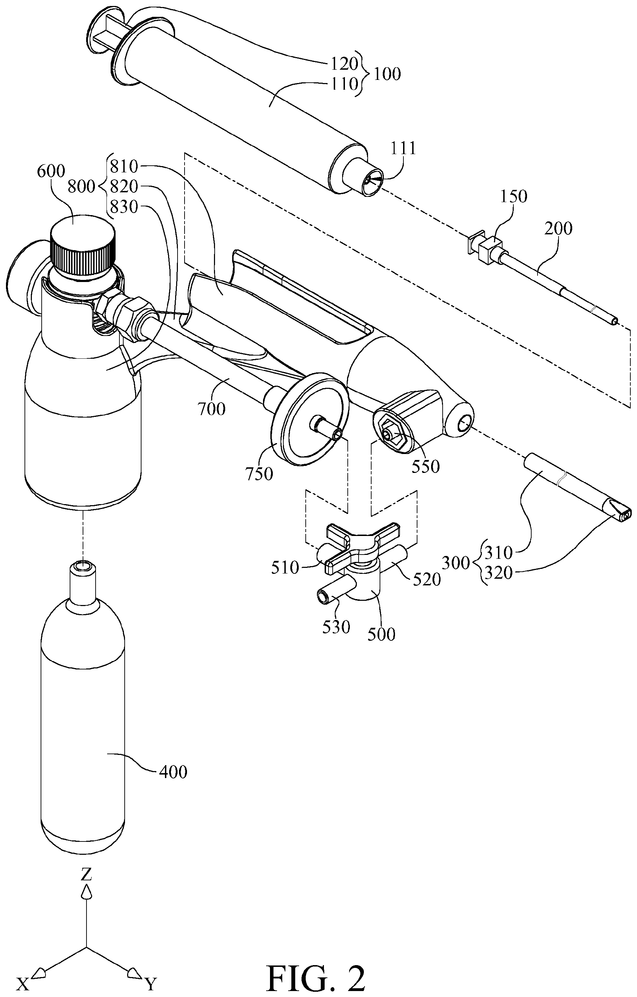

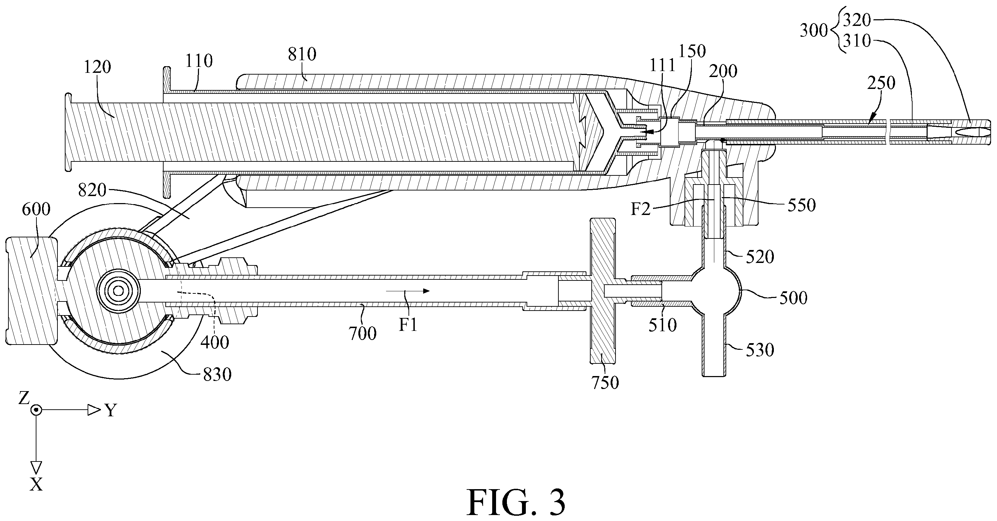

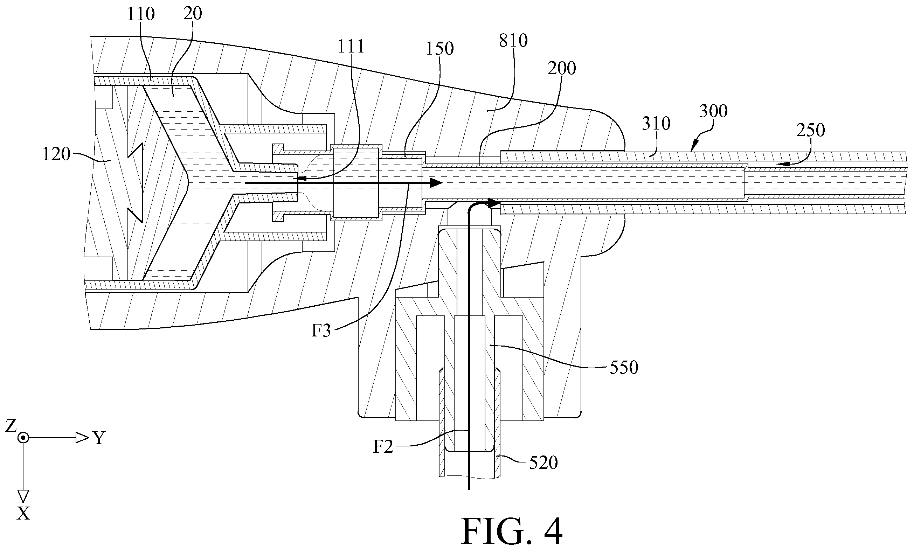

Please refer to FIGS. 1 to 4. FIG. 1 is a perspective view of a surgical device 10 according to a first embodiment of the disclosure. FIG. 2 is an exploded view of the surgical device 10 in FIG. 1. FIG. 3 is a top cross-sectional view of the surgical device 10 in FIG. 1. FIG. 4 is a partially enlarged side cross-sectional view of the surgical device 10 in FIG. 1.

In this embodiment, the surgical device 10 is configured to spray a flowable material 20 cooled by a coolant (not shown). The surgical device 10 may be disposable. The flowable material 20 is, for example, a thermosensitive hydrogel having a viscosity ranging from 0.001 poise Pa · s to 3000 Pa · s. In other embodiments, the flowable material may be cross-linkable polymer solutions. In other embodiments, the flowable material may include chemotherapy, DNA, RNA, nanomolecules, viruses, or immunotherapy.

In this embodiment, the surgical device 10 includes, for example, a syringe 100, a first inner tube 200, an outer tube 300, a container 400, a first valve 500, a second valve 600, a connecting tube 700, a filter 750 and an assembling component 800.

The syringe 100 includes, for example, a body 110 and a plunger 120 movably disposed in the body 110. The body 110 has an outlet 111 and is configured to accommodate the flowable material 20.

The first inner tube 200 is disposed on an end of the body 110 of the syringe 100 via, for example, a joint assembly 150. The first inner tube 200 is in fluid communication with the outlet 111 via, for example the joint assembly 150. In addition, for example, the first inner tube 200 is made of metal, such as steel.

The outer tube 300 is disposed on the syringe 100 and surrounds the first inner tube 200. A cooling channel 250 is formed between the first inner tube 200 and the outer tube 300. In addition, the first inner tube 200 is made of, for example, plastic or silicon.

The container 400 is in fluid communication with the cooling channel 250 and configured to accommodate the coolant. In this embodiment, the container 400 is, for example, a cartridge. The coolant is, for example, a gaseous coolant, such as gaseous carbon dioxide. Further, for example, the container 400 is configured to store compressed carbon dioxide, and thus the coolant that is the carbon dioxide released from the container 400 can effectively cool the flowable material 20. However, the disclosure is not limited to the type of the coolant. In other embodiments, the coolant may be gaseous nitrogen or air.

The cooling channel 250 in fluid communication with the container 400 is formed between the first inner tube 200 and the outer tube 300, and the first inner tube 200 is in fluid communication with the outlet 111 of the syringe 100. Thus, the coolant in the container 400 is allowed to flow into the cooling channel 250 to cool the flowable material 20 flowing into the first inner tube 200 from the outlet 111 of the syringe 100. In this way, the flowable material 20 is cooled to a constant low temperature to maintain fluidity, thereby facilitating the spraying of the flowable material 20. Specifically, the first inner tube 200 may be maintained at a low temperature within the range of -20 ° C to 25 ° C, to keep the flowable material 20 at such constant low temperature.

In this embodiment, the first valve 500 is, for example, a 3-way valve, and has a first port 510, a second port 520 and a third port 530. The first port 510 is in fluid communication with the container 400. The second port 520 is in fluid communication with the cooling channel 250 via, for example, a joint assembly 550. The third port 530 is exposed to the outside.

The second valve 600 is, for example, a switch valve, and is in fluid communication with the container 400 and the connecting tube 700. The second valve 600 is in fluid communication with the first port 510 via the connecting tube 700. The second valve 600 is functioned as a safety apparatus to ensure safe operation. In detail, the second valve 600 may be closed constantly. Before the surgery starts, the second valve 600 may be opened to release the coolant that is compressed. When the compressed coolant is released, the coolant will flow to the first valve 500 via the connecting tube 700, and the first port 510 and the third port 530 may be opened. In this way, the coolant flows to the outside via the third port 530, thereby generating a sound acting as a reminder of functional operation of the second valve 600. Then, the third port 530 may be closed and the coolant is allowed to flow to the cooling channel 250 via the second port 520 to start the spraying process of the flowable material 20, which will be described in detail later.

In addition, in this embodiment, the connecting tube 700 is in fluid communication with the first port 510 via the filter 750. The filter 750 is, for example, a filter core in 0.22 uL porosity. Thus, the particle or microorganism may be removed from the coolant.

Please refer to FIG. 2. In this embodiment, the assembling component 800 includes a first barrel part 810, a connecting part 820 and a second barrel part 830. The connecting part 820 connects the first barrel part 810 and the second barrel part 830. The body 110 of the syringe 100 is disposed in the first barrel part 810 and the container 400 is disposed in the second barrel part 830. Thus, the assembling component 800 facilitates the assembly of the syringe 100 and the container 400. In addition, the joint assembly 150 and the outer tube 300 are disposed in the first barrel part 810 via, for example, tight fit. Thus, the assembling component 800 facilitates the joint assembly 150 and the outer tube 300 to be disposed on the syringe 100.

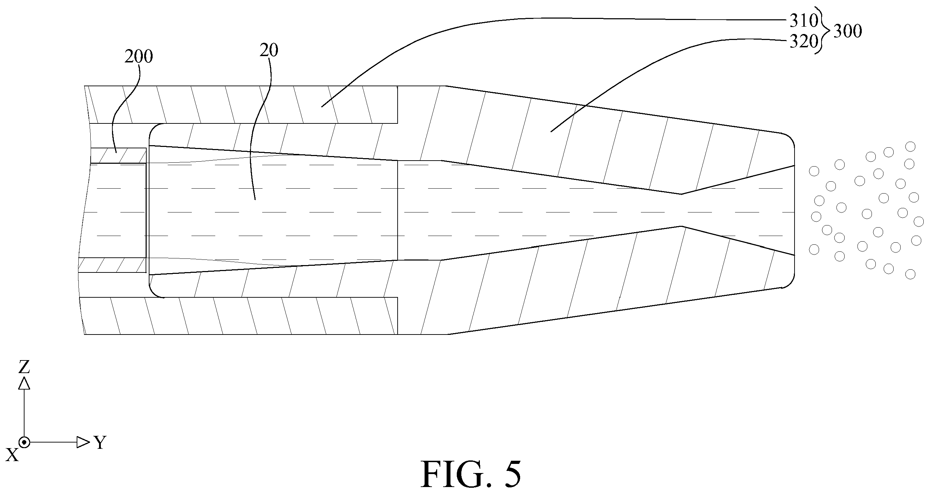

Furthermore, please refer to FIGS. 4 and 5. FIG. 5 is a side cross-sectional view showing a mixing portion of the surgical device in FIG. 1. In this embodiment, the outer tube 300 includes a cooling portion 310 and a mixing portion 320. The cooling portion 310 and the first inner tube 200 form the cooling channel 250. The mixing portion 320 is connected to an end of the cooling portion 310 away from the syringe 100, and has a mixing channel 321. The mixing channel 321 is in fluid communication with the cooling channel 250 and configured to mix the flowable material 20 and the coolant. When the flowable material 20 and the coolant are mixed, the flowable material 20 is atomized. In addition, in this embodiment, the mixing portion 320 tapers away from the cooling portion 310, and thus the mixture of the flowable material 20 and the coolant is facilitated, thereby facilitating the atomization of the flowable material 20.

Please refer to FIGS. 3 to 5. After the functional operation of the second valve 600 is reminded by the coolant flows to the outside via the third port 530, the third port 530 is closed, the first port 510 and the second port 520 is opened, and the spraying process of the flowable material 20 can starts. In detail, the coolant released from the container 400 flows to the first valve 500 via the second valve 600, the connecting tube 700 and the filter 750 along a flowing direction F1, and then flows to the cooling channel 250 via the second port 520 along a flowing direction F2. Also, the flowable material 20 is pushed by the plunger 120 and thus is injected into the first inner tube 200 via the outlet 111 along a flowing direction F3. Then, the flowable material 20 and the coolant flow to the mixing channel 321 and are mixed, thereby allowing the flowable material 20 to be atomized and sprayed to the affected part.

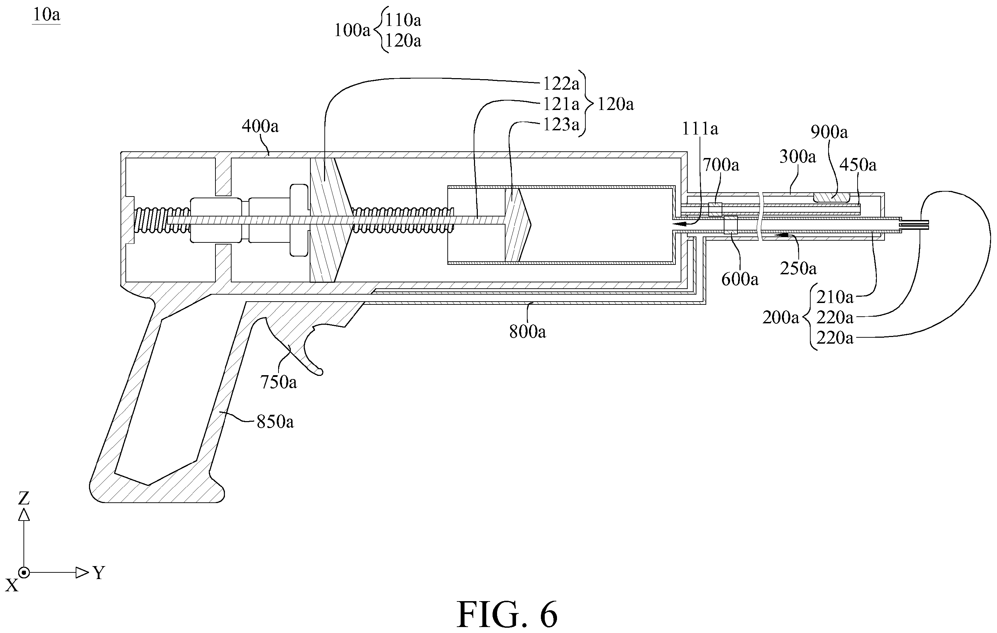

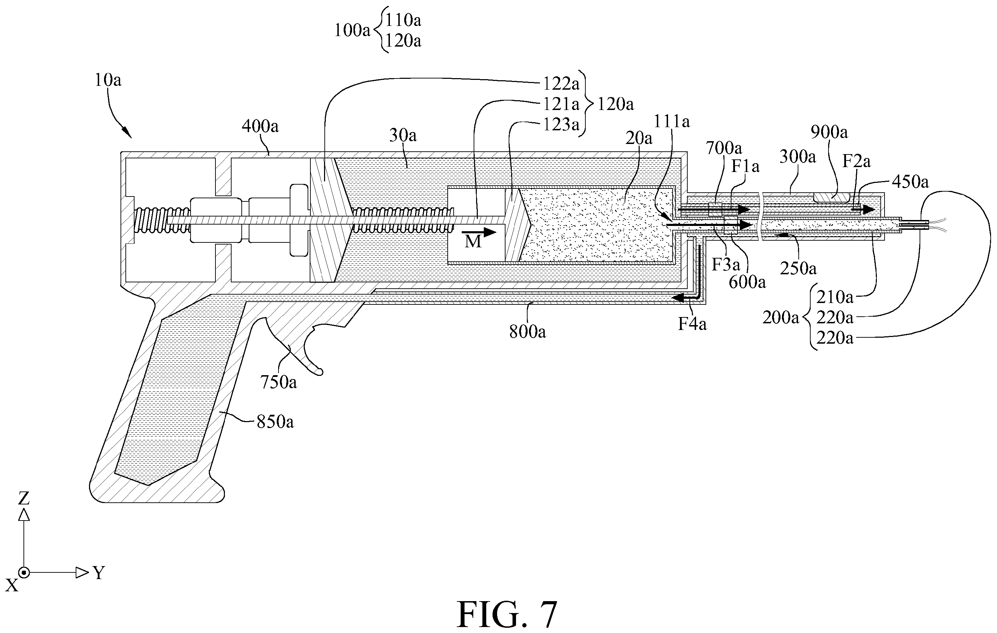

Please refer to FIGS. 6 and 7. FIGS. 6 and 7 are side cross-sectional views of a surgical device 10a according to a second embodiment of the disclosure. In this embodiment, the surgical device 10a is configured to spray a flowable material 20a cooled by a coolant 30a. The surgical device 10a may be disposable. The flowable material 20a is, for example, a thermosensitive hydrogel having a viscosity ranging from 0.001 poise Pa · s to 3000 Pa · s. In other embodiments, the flowable material may be cross-linkable polymer solutions.

In this embodiment, the surgical device 10a includes, for example, a syringe 100a, a first inner tube 200a, an outer tube 300a, a container 400a, a second inner tube 450a, a first valve 600a, a second valve 700a, a trigger 750a, a recycling tube 800a, and a recycling chamber 850a.

The syringe 100a includes, for example, a body 110a and a plunger 120a. The plunger 120a includes, for example, a rod 121a, a first piston 122a and a second piston 123a. The first piston 122a and the second piston 123a are disposed on the rod 121a and spaced part from each other. The body 110a is disposed in the container 400a and has an opening 111a. The first piston 122a is movably disposed in the container 400a. The second piston 123a is movably disposed in the body 110a. The cross-sectional area of the first piston 122a is, for example, larger than that of the second piston 123a. The body 110a is configured to accommodate the flowable material 20a.

The first inner tube 200a is disposed on an end of the body 110a of the syringe 100a. The first inner tube 200a is in fluid communication with the outlet 111a. In addition, for example, the first inner tube 200a is made of metal, such as steel.

The outer tube 300a is disposed on the syringe 100a and surrounds the first inner tube 200a. A cooling channel 250a is formed between the first inner tube 200a and the outer tube 300a. In addition, the first inner tube 200a is made of, for example, plastic or silicon.

The container 400a is in fluid communication with the cooling channel 250a and configured to accommodate the coolant 30a. In this embodiment, the coolant 30a is, for example, a liquid coolant, such as water. The coolant 30a may be previously cooled to a desired temperature before being stored in the container 400a.

The cooling channel 250a in fluid communication with the container 400a is formed between the first inner tube 200a and the outer tube 300a, and the first inner tube 200a is in fluid communication with the outlet 111a of the syringe 100a. Thus, the coolant in the container 400a is allowed to flow into the cooling channel 250a to cool the flowable material 20a flowing into the first inner tube 200a from the outlet 111a of the syringe 100a. In this way, the flowable material 20a is cooled to a constant low temperature to maintain fluidity, thereby facilitating the spraying of the flowable material 20a. Specifically, the first inner tube 200a may be maintained at a low temperature within the range of -10 ° C to 25 ° C, to keep the flowable material 20a at such constant low temperature. Note that due to the continuous flow of the coolant, the solidification of the coolant is nearly prevented from occurring.

The second inner tube 450a is disposed on the end of the body 110a of the syringe 100a. The first inner tube 200a and the second inner tube 450a are disposed on the same end of the body 110a. Two opposite ends of the second inner tube 450a are in fluid communication with the container 400a and the cooling channel 250a, respectively. The second inner tube 450a is located in the cooling channel 250a. In addition, the second inner tube 450a is made of, for example, plastic or silicon. Note that in this embodiment, the first inner tube 200a is not in fluid communication with the second inner tube 450a and the outer tube 300a. That is, in this embodiment, the flowable material 20a and the coolant 30a are not mixed.

In this embodiment, the first piston 122a is located farther away from the outer tube 300a than the second piston 123a. The first valve 600a is, for example, a block valve, and disposed in the first inner tube 200a. The second valve 700a is, for example, a block valve, and disposed in the second inner tube 450a. Thus, the temperature of the flowable material 20a is prevented from being significantly increased when the surgical device 10 is in contact with the affected part, which will be described in detail later.

The trigger 750a is movably disposed on the container 400a and sticks out of the container 400a. The rod 121a is fixed to a side of the trigger 750a. The trigger 750a sticking out of the container 400a is configured to move the rod 121a, and thus allows the rod 121a to be moved in a convenient manner.

The recycling chamber 850a is in fluid communication with the cooling channel 250a via the recycling tube 800a. The recycling chamber 850a is disposed on a side of the container 400a. The recycling chamber 850a and the trigger 750a may be disposed on the same side of the container 400a.

In this embodiment, the first inner tube 200a includes a covered portion 210a and a plurality of exposed portions 220a in fluid communication with the covered portion 210a. The exposed portions 220a branch from the cover portion 210a and stick out of the outer tube 300a. The covered portion 210a is disposed on the end of the body 110a. The covered portion 210a and the outer tube 300a form the cooling channel 250a. The exposed portions 220a are configured to spray the flowable material 20a.

In this embodiment, the surgical device 10a may further include a temperature sensor 900a disposed on the outer tube 300a to detect the temperature of the coolant.

Please refer to FIG. 7 for the spraying process performed by the surgical device 10a. The trigger 750a is moved to force the rod 121a to be moved along a moving direction M. At this time, the first valve 600a is closed and the second valve 700a is opened. Thus, the coolant 30a flows into the second inner tube 450a by being pushed by the first piston 122a along a flowing direction Fla. The coolant 30a flowing into the second inner tube 450a then flows into the outer tube 300a along a flowing direction F2a. Then, the first valve 600a is opened and the flowable material 20a flows into the first inner tube 200a by being pushed by the second piston 123a along a flowing direction F3a. Due to such mechanism, the coolant 30a is ensured to be filled in the outer tube 300a before the flowable material 20a is filled in the first inner tube 200a, thereby preventing the temperature of the flowable material 20a from significantly increased when the surgical device 10 is in contact with the affected part.

In addition, When the outer tube 300a is filled with the coolant 30a, the coolant 30a may flow to the recycling chamber 850a by the recycling tube 800a along a flowing direction F4a. Thus, the coolant 30a that has absorbed the heat from the flowable material 20a is recycled into the recycling chamber 850a, and thus the low temperature of the flowable material 20a is ensured.

Note that in this embodiment, the body 110a is disposed in the container 400a, and the plunger 120a includes the rod 121a, the first piston 122a and the second piston 123a. Thus, the injection of the flowable material 20a and the injection of the coolant 30a may be performed by the same plunger 120a. However, in other embodiments, the body may be disposed outside the container, and there may be separate plungers respectively disposed in the body and the container, thereby allowing the injection of the flowable material and the injection of the coolant to be performed independently.

According to the surgical device discussed above, the cooling channel in fluid communication with the container is formed between the first inner tube and the outer tube, and the first inner tube is in fluid communication with the outlet of the syringe. Thus, the coolant in the container is allowed to flow into the cooling channel to cool the flowable material flowing into the first inner tube from the outlet of the syringe. In this way, the flowable material is cooled to a constant low temperature to maintain fluidity, thereby facilitating the spraying of the flowable material.

It will be apparent to those skilled in the art that various modifications and variations can be made to the present disclosure. It is intended that the specification and examples be considered as exemplary embodiments only, with a scope of the disclosure being indicated by the following claims and their equivalents.

## Claims

1. A surgical device, configured to spray a flowable material cooled by a coolant and comprising:
a syringe, having an outlet and configured to accommodate the flowable material;
a first inner tube, disposed on an end of the syringe and in fluid communication with the outlet;
an outer tube, disposed on the syringe and surrounds the first inner tube, wherein a cooling channel is formed between the first inner tube and the outer tube; and
a container, in fluid communication with the cooling channel and configured to accommodate the coolant.

2. The surgical device according to claim 1, wherein the outer tube includes a cooling portion and a mixing portion, the cooling portion and the first inner tube form the cooling channel, the mixing portion is connected to an end of the cooling portion away from the syringe and has a mixing channel, the mixing channel is in fluid communication with the cooling channel and configured to mix the flowable material and the coolant.

3. The surgical device according to claim 2, wherein the mixing portion tapers away from the cooling portion.

4. The surgical device according to claim 1, further comprising a first valve, wherein the first valve has a first port, a second port and a third port, the first port is in fluid communication with the container, the second port is in fluid communication with the cooling channel, and the third port is exposed to the outside.

5. The surgical device according to claim 4, further comprising a second valve and a connecting tube, wherein the container is in fluid communication with the second valve, and the second valve in in fluid communication with the first port via the connecting tube.

6. The surgical device according to claim 5, further comprising a filter, wherein the connecting tube is in fluid communication with the first port via the filter.

7. The surgical device according to claim 1, further comprising an assembling component, wherein the assembling component comprises a first barrel part, a connecting part and a second barrel part, the connecting part connects the first barrel part and the second barrel part, the syringe is disposed in the first barrel part and the container is disposed in the second barrel part.

8. The surgical device according to claim 1, wherein the first inner tube is made of metal.

9. The surgical device according to claim 1, wherein the outer tube is made of plastic or silicon.

10. The surgical device according to claim 1, further comprising a second inner tube, wherein the second inner tube is disposed on the end of the syringe and in fluid communication with the container and the cooling channel, and the second inner tube is located in the cooling channel.

11. The surgical device according to claim 10, wherein the syringe comprises a body and a plunger, the plunger comprises a rod, a first piston and a second piston, the first piston and the second piston are disposed on the rod and spaced part from each other, the body is disposed in the container, the opening is located on the body, the first piston is movably disposed in the container, and the second piston is movably disposed in the body.

12. The surgical device according to claim 11, wherein the first piston is located farther away from the outer tube than the second piston.

13. The surgical device according to claim 12, further comprising a first valve and a second valve, wherein the first valve is disposed in the first inner tube, and the second valve is disposed in the second inner tube.

14. The surgical device according to claim 11, further comprising a trigger, wherein the trigger is movably disposed on the container and sticks out of the container, and the rod is fixed to a side of the trigger.

15. The surgical device according to claim 10, further comprising a recycling tube and a recycling chamber, wherein the recycling chamber is in fluid communication with the cooling channel via the recycling tube, and the recycling chamber is disposed on a side of the container.

16. The surgical device according to claim 10, wherein the first inner tube comprises a covered portion and a plurality of exposed portions in fluid communication with the covered portion, the plurality of exposed portions branch from the cover portion and stick out of the outer tube, the covered portion is disposed on the end of the syringe, and the covered portion and the outer tube form the cooling channel.

17. The surgical device according to claim 1, wherein the flowable material includes chemotherapy, DNA, RNA, nanomolecules, viruses, or immunotherapy.
